# EUROPEAN PATENT APPLICATION

(11) **EP 4 261 468 A1**
(43) Date of publication of application: **18.10.2023**
(21) Application number: 22728024.5
(22) Date of filing: 04.01.2022
(51) Int. Cl.: F24F 8/50, F24F 8/108, F24F 8/22, A61L 9/20

(54) **MIXED OUTDOOR AND INDOOR AIR PURIFIER**

(30) Priority: 04.12.2020 ES 202032634 U
(71) Applicant: Sánchez Campos, Roberto, 41010 Sevilla (ES)
(72) Inventor: Sánchez Campos, Roberto, 41010 Sevilla (ES)
(74) Representative: Jiménez Diaz, Rafael
(86) International application number: PCT/ES2022/070002
(87) International publication number: WO 2022/117904

(57) **Abstract**

The invention consists of a purifier equipment container (1) with an air inlet port (2), an air outlet port (4), an extractor fan and a set of filters, along with an ultraviolet emitting device (10), comprising, upstream of the air inlet port (2), a stretch of a mixing duct (3) transporting therein a mix of air, part thereof from the interior of the dwelling and part from the exterior, from outdoors, and further comprising a flexible duct (12) between the extractor fan (11) and the air outlet port (4), to which there is connected a humidifier device (13) that sprays the purified air with a scented disinfectant fluid (14) in atomised droplets with a citrus fruit scent.

## Description

### OBJECT OF THE INVENTION

The present invention relates to an air purifier for the consumption of filtered air for people and animals, allowing the consumer to inhale purified and disinfected air against viruses, bacteria, scents and particles that may be contained in the air of the room where it is located.

In addition to purifying and disinfecting the air in the room, it renews it by supplying air from the exterior, so that the air never becomes very stale.

Due to the situation of the occurrence of the COVID-19 virus and other similar ones, it is essential to use air purifier equipment in any room where people and animals live, thus creating a new essential household appliance for everyday life.

The industrial application of this invention is within the sector of purification and disinfection equipment and household appliances, and more specifically, mixed outdoor and indoor air purifiers.

### BACKGROUND OF THE INVENTION

To purify the air, purifier equipment is required, and there are different models and types on the market today.

However, none of them meets all the recommendations made by the World Health Organization (WHO), the Ministry of Health, the Ministry for the Ecological Transition and the Demographic Challenge, as well as the Institute for Energy Diversification and Saving (IDEA), in the same device, the object of the present invention being to complete said air purifying need, adding the disinfection section in the air treatment process.

On the other hand, current air purifier equipment has a high cost, making it very difficult to acquire same for a sector of the population with limited financial resources. And in those cases that they acquire it, manufacturers add an extra responsibility, obliging the user to carry out maintenance work, such as cleaning, replacement of filters and other consumables.

### DESCRIPTION OF THE INVENTION

The mixed indoor and outdoor air purifier object of the present invention is made up of a piece of equipment designed to be fixed to the wall by means of fastening or anchoring, and houses therein the following elements and devices:
A suction air port at the inlet, for connecting the purifier equipment to a stretch of duct on site, transporting therein a mix of air, part thereof from the interior of the dwelling and part from the exterior, from outdoors.

A supply air port at the outlet, for connecting the purifier equipment to a stretch of duct on site, transporting therein the already filtered and disinfected air, to connect with an air conditioning unit with which the function thereof is combined, absorbing said purified air, and expelling it into the room to be air conditioned.

A set of filters: a mesh filter, a foam filter, an activated carbon filter, a HEPA (High Efficiency Particulate Air) filter, and a photocatalytic filter, for all types of particles, from smaller solids dissolved in the air (lint, leaves, hair, insects, etc.), allergens, smoke, microscopic particles, and in the case of the photocatalytic filter, it comprises a mesh impregnated with titanium dioxide that reacts with light from an ultraviolet emitting device, breaking the molecular structure of viruses and bacteria.

An extractor fan, which sucks the air from the inlet air port, passes it through the filters, and expels it into the next compartment of the equipment up to the air outlet of the equipment object of the present invention.

A flexible duct between the extractor fan and the air outlet port, to which a humidifier device is connected that sprays the purified air with disinfectant fluid in atomised droplets, said fluid also having a citrus fruit scent to simultaneously combat potential bad odours that derive from human and animal activity.

A manual control and regulation system with data input means such as a keyboard, wherein the operation of the equipment, such as switching on, switching off, speed, timer, etc., is managed, and it further has wireless control means for control by means of infrared remote control, or by means of a smartphone or similar device via Wi-Fi or Bluetooth connection.

All the equipment works connected to the electrical system in the dwelling.

### BRIEF DESCRIPTION OF THE DRAWINGS

For a better understanding of the present description, drawings are attached that depict a preferred embodiment of the present invention:

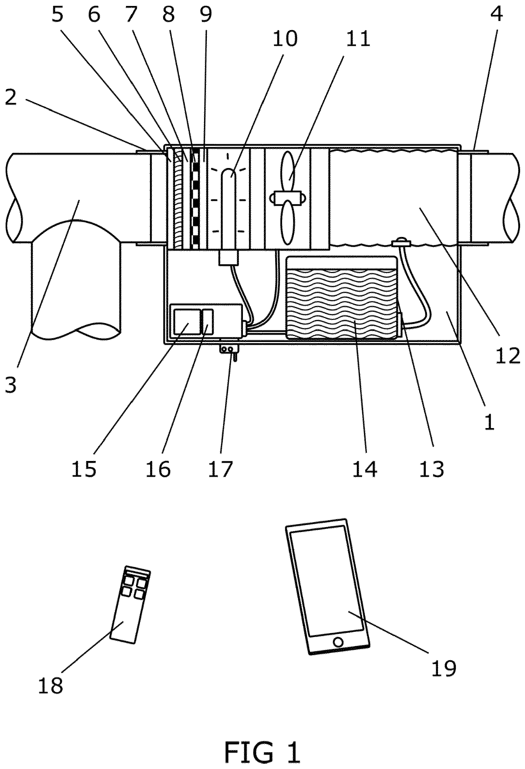

Figure 1 shows a schematic view of an exemplary model of a mixed outdoor and indoor air purifier object of the present invention.

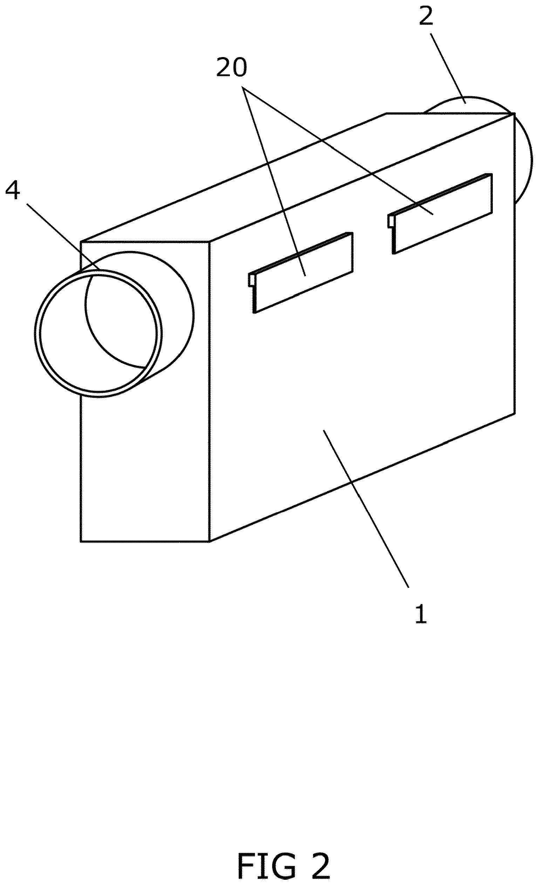

Figure 2 shows a rear conventional perspective view of the mixed outdoor and indoor air purifier object of the present invention.

The reference numbers appearing in said figures correspond to the following constituent elements of the invention:
- 1.: Purifier equipment container
- 2.: Air inlet port
- 3.: Mixing duct
- 4.: Air outlet port
- 5.: Mesh filter
- 6.: Foam filter
- 7.: Activated carbon filter
- 8.: HEPA filter
- 9.: Photocatalytic filter
- 10.: UV light emitter
- 11.: Extractor fan
- 12.: Flexible duct
- 13.: Humidifier
- 14.: Scented disinfectant fluid
- 15.: Control and regulation system
- 16.: Data input and output means
- 17.: Wireless control means
- 18.: Remote control
- 19.: Smartphone or similar electronic device
- 20.: Fixing means

### DESCRIPTION OF A PREFERRED EMBODIMENT

A preferred embodiment of the mixed outdoor and indoor air purifier object of the present invention, with reference to the reference numbers, can be based on a purifier equipment container (1) comprising the following elements and devices:
An air inlet port (2) for connecting the purifier equipment with a stretch of a mixing duct (3) transporting therein a mix of air, part thereof from the interior of the dwelling and part from the exterior, from outdoors.

An air outlet port (4) for connecting the purifier equipment with an air conditioning unit, which expels the treated air into the room to be air conditioned.

A set of filters: a mesh filter (5), a foam filter (6), an activated carbon filter (7), a HEPA filter (8), and a photocatalytic filter (9), along with an ultraviolet emitting device (10).

An extractor fan (11), which sucks the air from the air inlet port (2), passes it through the filters (5, 6, 7, 8, 9), and expels it into the next compartment of the equipment up to the air outlet (4).

A flexible duct (12) between the extractor fan (11) and the air outlet port (4), to which there is connected a humidifier device (13) that sprays the purified air with a scented disinfectant fluid (14) in atomised droplets with a citrus fruit scent.

A manual control and regulation system (15) with data input and output means (16) such as a keyboard, wherein the operation of the equipment, such as switching on, switching off, speed, timer, etc., is managed, and it further has wireless control means (17) for control by means of infrared remote control (18), or by means of a smartphone or similar device (19) via Wi-Fi or Bluetooth connection.

The equipment is fixed to the wall by fastening means (20) and is connected to the electrical system in the dwelling.

## Claims

1. A mixed outdoor and indoor air purifier, consisting of a purifier equipment container (1) with an air inlet port (2), an air outlet port (4), an extractor fan and a set of filters: a mesh filter (5), a foam filter (6), an activated carbon filter (7), a HEPA filter (8), and a photocatalytic filter (9), along with an ultraviolet emitting device (10), **characterised in that** it comprises, upstream of the air inlet port (2), a stretch of a mixing duct (3) transporting therein a mix of air, part thereof from the interior of the dwelling and part from the exterior, from outdoors, and further comprising a flexible duct (12) between the extractor fan (11) and the air outlet port (4), to which there is connected a humidifier device (13) that sprays the purified air with a scented disinfectant fluid (14) in atomised droplets with a citrus fruit scent, and further comprising a manual control and regulation system (15) with data input and output means (16), wherein the operation of the equipment, such as switching on, switching off, speed, timer, etc., is managed.

2. The mixed outdoor and indoor air purifier according to claim 1, wherein the control and regulation system (15) comprises wireless control means (17) for control by means of an infrared remote control (18), or by means of a smartphone or similar device (19) via Wi-Fi or Bluetooth connection.

3. The mixed indoor and outdoor air purifier according to claim 1, wherein the purifier equipment container (1) comprises fixing means (20) to anchor same to a support or wall.
